(19) Europäisches Patentamt
European Patent Office
Office européen des brevets

(11) Veröffentlichungsnummer: **0 426 055 A2**

# (12) EUROPÄISCHE PATENTANMELDUNG

(21) Anmeldenummer: **90120653.2**

(22) Anmeldetag: **27.10.90**

(51) Int. Cl.[5]: **C08G 63/06**, A61K 47/34, A61K 9/70, A61L 17/00, A01N 25/10

(30) Priorität: **31.10.89 DE 3936191**

(43) Veröffentlichungstag der Anmeldung: **08.05.91 Patentblatt 91/19**

(84) Benannte Vertragsstaaten: **AT BE CH DE DK ES FR GB GR IT LI LU NL SE**

(71) Anmelder: **BOEHRINGER INGELHEIM KG**
**W-6507 Ingelheim am Rhein(DE)**
(84) **BE CH DE DK ES FR GR IT LI LU NL SE AT**

Anmelder: **BOEHRINGER INGELHEIM INTERNATIONAL G.M.B.H.**
**W-6507 Ingelheim am Rhein(DE)**
(84) **GB**

(72) Erfinder: **Hess, Joachim, Dr. Dipl.-Chem.**
**Mozartstrasse 8**
**W-6530 Bingen(DE)**
Erfinder: **Müller, Klaus Robert, Dipl.-Ing.-Dipl.-Chem.**
**Am Rüsterbaum 9**
**W-6507 Ingelheim am Rhein(DE)**

(54) **Neue Copolymere aus Milchsäure und Weinsäure, ihre Herstellung sowie ihre Verwendung.**

(57) Die Erfindung betrifft neue Copolymere aus Milchsäure und Weinsäure, ihre Herstellung und Verwendung.

EP 0 426 055 A2

## NEUE COPOLYMERE AUS MILCHSÄURE UND WEINSÄURE, IHRE HERSTELLUNG SOWIE IHRE VERWENDUNG

Die Erfindung betrifft neue bioabbaubare Copolymere aus Milchsäure und Weinsäure, ihre Herstellung sowie ihre Verwendung.

Bioabbaubar bzw. resorbierbar bedeutet im Sinne der vorliegenden Erfindung, sofern nicht anders angegeben, daß das Polymer unter den im menschlichen oder tierischen Körper herrschenden physiologischen Bedingungen im Laufe der Zeit aufgelöst und zu toxikologisch unbedenklichen Produkten abgebaut wird.

Der Entwicklung von bioabbaubaren Copolymerisaten, die Milchsäureeinheiten enthalten, ist in den vergangenen Jahren verstärkte Aufmerksamkeit gewidmet worden. Die besondere Rolle der Milchsäure begründet sich dabei -besonders im Hinblick auf die Verwendung Milchsäure enthaltender Copolymerisate für medizinische Zwecke -auf die gute physiologische Verträglichkeit, durch die sich die Milchsäure auszeichnet.

Während im Rahmen der Entwicklung derartiger bioabbaubarer Copolymerisate für die unterschiedlichsten Anwendungszwecke, Milchsäure bzw. Lactid mit einer Vielzahl von diversen Monomeren cokondensiert bzw. copolymerisiert wurde, sind Copolymerisate, die Milchsäure- und Weinsäureeinheiten enthalten, bislang unbekannt.

Dabei umfaßt der Begriff "Milchsäure" - wie er in der vorliegenden Anmeldung verwendet wird - D-Milchsäure und L-Milchsäure sowie Mischungen davon. Desgleichen umfaßt der Begriff Weinsäure (Dihydroxybernsteinsäure), die beiden Enantiomeren (+)-Weinsäure [(2R,3R)-(+)-Weinsäure] sowie (-)-Weinsäure [(2s,3s)-(-)-Weinsäure] und das Racemat (Traubensäure) sowie die optisch inaktive meso-Weinsäure und deren Mischungen.

Während aus der Polykondensation von Milchsäure mit -den üblicherweise eingesetzten -α-Hydroxycarbonsäuren im allgemeinen nur unverzweigte, lineare Polyester hergestellt werden können, resultieren aus der Cokondensation von Milchsäure und Weinsäure Cokondensationsprodukte mit verzweigten Strukturen.

Auf Milchsäure basierende Polycokondensate, die einen linearen Aufbau aufweisen, besitzen verhältnismäßig geringe Molekulargewichte. So weisen Copolymere aus Milchsäure und Glykolsäure ein Molekulargewicht auf, das maximal im Bereich von 3500 bis 4000 liegt, was sich in den Werten der inhärenten Viskosität, die in einem Bereich von ca. 0,1 bis 0,15 dl/g liegen, widerspiegelt. Dagegen zeigen die erfindungsgemäßen Polykondensationsprodukte aus Milchsäure und Weinsäure Werte der inhärenten Viskosität von größer 0.5 dl/g, obwohl die Polykondensation nur einen Bruchteil der Zeit beansprucht, die für Polykondensationen von Milchsäure und Glykolsäure benötigt wird.

Die erfindungsgemäßen Copolymere werden im wesentlichen aus Einheiten der Formel I

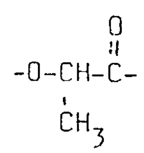

(I)

und Einheiten der Formel II

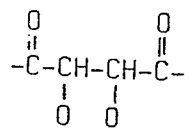

(II)

aufgebaut. Dabei können - abhängig vom jeweiligen Umsetzungsgrad in beiden Einheiten freie Carboxyl- oder Hydroxylgruppen in geringen oder größeren Anteilen vorliegen.

In den Polykondensationsprodukten der vorliegenden Erfindung liegt das Verhältnis von Milchsäure-Einheiten zu Weinsäure-Einheiten in einem Bereich von 1 zu 2 bis 60 zu 1. Bevorzugt wird ein Bereich von 1 zu 1 bis 50 zu 1, wobei der Bereich von 5 zu 1 bis 30 zu 1 besonders bevorzugt wird.

Die erfindungsgemäßen Copolymerisate weisen Werte der inhärenten Viskosität (gemessen in 0,1

prozentiger Lösung in Aceton bei 20° C) auf, die im Bereich von 0.2 bis 1 dl/g, bevorzugt 0.2 bis 0.8 dl/g liegen. Besonders bevorzugt werden Copolymerisate, deren inhärente Viskosität in einem Intervall von 0.2 bis 0.6 dl/g liegen.

Ferner können die erfindungsgemäßen Cokondensate kleinere Mengen von Einheiten einer weiteren Hydroxycarbonsäure enthalten oder es können weitere Monomere aus der Gruppe der Lactone oder Lactide in die Kondensationsreaktion mit eingeführt werden.

Die erfindungsgemäßen Produkte sind in den meisten gängigen organischen Lösungsmitteln unlöslich oder quellen in diesen stark auf, was auf eine Vernetzung im Polymeren hindeutet.

Im Vergleich zu den bisher bekannten Polymeren enthalten die erfindungsgemäßen Copolymerisate einen hohen Anteil an freien Carboxylgruppen. Dadurch bedingt besitzen sie die Eigenschaft, daß sich ihre Wasserlöslichkeit über den pH-Wert steuern läßt.

Die erfindungsgemäßen bioabbaubaren Polyester weisen somit neuartige physikalische und chemische Eigenschaften auf, die sie für eine Vielzahl von interessanten Anwendungsformen als geeignet erscheinen lassen, von denen im folgenden lediglich einige beispielhaft aufgeführt sind:

1) Die erfindungsgemäßen Polykondensationsprodukte eignen sich als Träger (Matrix) oder Vorrichtungen zur Speicherung therapeutischer Mittel bzw. als Retardierungshilfsstoffe für die Verwendung in Wirkstoffreigabesystemen, bei denen eine kontrollierte Freigabe des Pharmazeutikums erzielt werden soll. Bei derartigen Wirkstoffreigabesystemen ist es erwünscht, daß sich das Polymer während oder nach der Freigabe des Arzneimittels auflöst und dabei keine unerwünschten oder gar pharmakologisch bedenklichen Rückstände im Gewebe hinterläßt.

Aufgrund ihrer pH-abhängigen Wasserlöslichkeit sind die erfindungsgemäßen Copolymerisate für eine derartige Anwendungsform besonders interessant, da sie durch diese Eigenschaft neben den bislang zur Verfügung stehenden und aus dem Stand der Technik bekannten Möglichkeiten eine weitere Möglichkeit erschließen, auf die Freigabecharakteristik Einfluß zu nehmen.

Verfahren, die erfindungsgemäßen Polykondensationsprodukte mit Wirkstoff(en) zu beladen, bzw. das Wirkstoffreigabesystem z.B. durch bestimmte Verfahrensmaßnahmen von vornherein so zu gestalten, daß eine bestimmte Freigabecharakteristik erzielt wird, sind aus dem Stand der Technik bekannt. - Dabei besitzen die erfindungsgemäßen Kondensationsprodukte zusätzlich den Vorteil, daß durch die hohe Zahl der im polymerisat vorhandenen freien Carboxylgruppen eine physikalische und/oder chemische Bindung geeigneter Wirkstoffe an die Matrix ermöglicht wird, ohne daß dazu weitere Veränderungen an dem Polymerisat notwendig wären.

So gestaltete Freigabesysteme können als Implantat wie auch für eine orale Aplikation oder für eine Verabreichung in Form eines Aerosols konzipiert werden.

Die absorbierbaren Polykondensationsprodukte können z.B. gemahlen und mit den gewünschten Wirkstoffen und gegebenenfalls weiteren Hilfsstoffen - wie z.B. Bindemitteln oder Geschmacksstoffen - zu Tabletten verpresst oder zu Dragées oder Pellets verarbeitet werden. Entsprechende Verfahren sind aus dem Stand der Technik bekannt. Auch bei dieser Anwendungsform verkörpert die pH-abhängige Wasserlöslichkeit der erfindungsgemäßen Copolymerisate einen besonderen Vorteil, da in Anbetracht der Tatsache, daß im Magen ein saures Milieu, im Darm jedoch ein alkalisches Milieu vorliegt eine gezielte Freigabe erreicht werden kann.

2) Die erfindungsgemäßen Copolymerisate eignen sich ferner zur Verwendung in Form resorbierbarer chirurgischer Hilfsmittel, die bei chirurgischen Eingriffen verwendet werden.

Die einzelnen Anwendungsmöglichkeiten derartiger Polymerisate sind aus dem Stand der Technik bekannt. Im allgemeinen werden diese Einsatzmöglichkeiten in erster Linie zum einen durch die mechanischen Eigenschaften und zum anderen durch das in vivo Abbauverhalten bestimmt. Beide Charakteristika lassen sich unter anderem durch die Zusammensetzung der jeweiligen Cokondensationsprodukte in einem weiten Bereich steuern.

Die Verwendung von resorbierbaren Polymeren erweist sich immer dann als besonders vorteilhaft, wenn - z.B. infolge einer erschwerten Zugänglichkeit - das Entfernen der chirurgischen Befestigungsvorrichtung (z.B. Klammern, orthopädische Stifte oder Schienen) nach Beendigung des Eingriffs mit einem hohen Arbeitsaufwand oder mit einem Gefahrenmoment verbunden wäre.

Aber auch im umgekehrten Falle - wenn also von vornherein die Absicht besteht, das chirurgische Hilfsmittel am Ende des Eingriffs zu entfernen erweist sich die Verwendung der erfindungsgemäßen Polykondensationsprodukte als vorteilhaft, da die bioabbaubaren Polymerisate im Gegensatz zu den nicht abbaubaren bei einem unbeabsichtigten Verbleib im Körper gewöhnlich ohne nachteilige Folgen absorbiert werden können und nicht zu Komplikationen führen. Als Beispiele seien u.a. Plastiken, Dentalpackungen oder Vorrichtungen zur Hämostasis genannt.

Erforderlichenfalls können die aus den erfindungsgemäßen Cokondensationsprodukten hergestellten

chirurgischen Hilfsmittel auch als Träger für Pharmazeutika - wie z.B. entzündungshemmende oder bakterizide Mittel -dienen oder z.B. mit einer einen Röntgenkontrast erzeugenden Substanz versehen werden.

3) Die oben erwähnte, steuerbare Wasserlöslichkeit der erfindungsgemäßen Cokondensate, eröffnet ferner die Möglichkeit, diese beispielsweise als Träger oder Behältnisse z.B. für Agrochemikalien, Herbizide oder Insektizide einzusetzen. Der verhältnismäßig hohe Gehalt an freien Carboxylgruppen ist auch bei dieser Anwendungsform mit dem Vorteil verbunden, daß auch - im Vergleich zu den therapeutischen Anwendungsformen - größere Mengen an Wirkstoff chemisch und/oder physikalisch an die Matrix gebunden werden können.

4) Die erfindungsgemäßen Polykondensationsprodukte können in einer weiteren Anwendungsform durch Erhöhen des Weinsäure-Anteils so gestaltet werden, daß sie sich äußerst leicht in Wasser auflösen. Diese Eigenschaft läßt sie für eine Verwendung in Form von Schutzfilmen oder Schutzfolien, die leicht und ohne Anwendung von anderen organischen oder anorganischen Lösungsmitteln entfernt werden sollen, als geeignet erscheinen. Dabei bringt die Verwendung der erfindungsgemäßen Polykondensate den großen Vorteil mit sich, daß das Polymerisationsprodukt selbst wie auch dessen Hydrolyseprodukte biologisch abbaubar sind, wodurch eine hohe umweltverträglichkeit gewährleistet ist.

Die erfindungsgemäßen Polymerisate können mit den aus dem stand der Technik bekannten Polykondensationsmethoden gegebenenfalls in Gegenwart eines Kondensationskatalysators hergestellt werden. Dabei können anstelle von Milchsäure bzw. deren Enantiomeren auch die entsprechenden Lactide als Ausgangsmaterial eingesetzt werden. Die Hinzufügung einer weiteren Hydroxycarbonsäure, eines Lactons oder Lactids kann dabei vor oder während der Reaktion erfolgen. Die nachfolgenden Beispiele sollen die Erfindung erläutern, ohne sie einzuschränken.

Beispiel 1

500 g L-Milchsäure (90 %-ig = 5.0 mol) werden in einer Rührapparatur, bestehend aus einem 1 L Sulfierkolben mit Rührer, Thermometer und Rückflußkühler, der als Dephlegmator (80° C) ausgelegt wird, mit 150.1 g L-Weinsäure (1.0 mol) vermengt. Das Reaktionsgemischwird unter Rühren und unter vermindertem Druck(Wasserstrahlvakuum) langsam auf ca. 170° C erwärmt, wobei das entstehende Reaktionswasser über den Dephlegmator abdestilliert wird. Die Polykondensationsreaktion wird so lange durchgeführt, bis die Viskosität der dabei entstehenden farblosen Produktschmelze so hohe Werte annimmt, daß ein Durchmischen mit Hilfe des Rührers nur noch schwer möglich ist.

Nach dem Austragen des Reaktionsproduktes werden die in der Tabelle 1 für die dort aufgeführten Zusammensetzungen von Milchsäure und Weinsäure angegebenen physikalischen und chemischen Daten bestimmt:

Tabelle 1:

| Beisp. Nr. | Verh. MS/WS[1)] | Reakt.-zeit/h | inh.Visk. dl/g | Säuregeh./%-COOH[2)] |
|---|---|---|---|---|
| 1 | 5/1 | 5.7 | 0.20 | 12.0-13.0 |
| 2 | 10/1 | 12.5 | 0.54 | 7.5-8.0 |
| 3 | 20/1 | 25.5 | 0.53 | 5.8 |
| 4 | 30/1 | 39.0 | 0.41 | 4.3 |

1) MS: L-Milchsäure
WS: L-Weinsäure
2) Gew.-%

## Ansprüche

1) Copolymer, dadurch gekennzeichnet, daß es Einheiten der Formel I

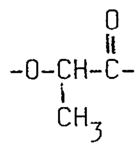

, (I)

und Einheiten der Formel II

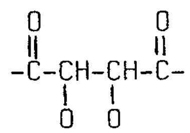

(II)

enthält.

2) Copolymer dadurch gekennzeichnet, daß es aus Milchsäure-und Weinsäureeinheiten besteht.

3) Copolymer nach Anspruch 1 oder 2, dadurch gekennzeichnet, daß es D-Milchsäure- und/oder L-Milchsäure-Einheiten enthält.

4) Copolymer nach einem der Ansprüche 1 bis 3, dadurch gekennzeichnet, daß es Einheiten der (2R, 3R)-(+)-Weinsäure und/oder der (2S,3S)-(-)-Weinsäure und/oder der meso-Weinsäure enthält.

5) Copolymer nach einem der Ansprüche 1 bis 4, dadurch gekennzeichnet, daß das Verhältnis von Milchsäure- zu Weinsäureeinheiten im Bereich von 1 zu 2 bis 60 zu 1 liegt.

6) Copolymer nach Anspruch 5, dadurch gekennzeichnet, daß das Verhältnis von Milchsäure- zu Weinsäure-Einheiten im Bereich von 1 zu 1 bis 50 zu 1 liegt.

7) Copolymer nach Anspruch 5, dadurch gekennzeichnet, daß das Verhältnis von Milchsäure- zu Weinsäure-Einheiten im Bereich von 5 zu 1 bis 30 zu 1 liegt.

8) Copolymer nach einem der Ansprüche 1 bis 7, dadurch gekennzeichnet, daß es einen Säuregehalt von über 4,3 % an freien Carboxylgruppen besitzt.

9) Copolymer nach einem der Ansprüche 1 bis 7, dadurch gekennzeichnet, daß es Werte der inhärenten Viskosität, die im Bereich von 0.2 bis 1 dl/g liegen - gemessen in 0,1 prozentiger Lösung in Aceton bei 20° C, aufweist.

10) Copolymer nach Anspruch 9, dadurch gekennzeichnet, daß es Werte der inhärenten Viskosität, die im Bereich von 0.2 bis 0.8 dl/g liegen - gemessen in 0,1 prozentiger Lösung in Aceton bei 20° C, aufweist.

11) Copolymer nach Anspruch 9, dadurch gekennzeichnet, daß es Werte der inhärenten Viskosität, die im Bereich von 0.2 bis 0.6 dl/g liegen - gemessen in 0,1 prozentiger Lösung in Aceton bei 20° C, aufweist.

12) Verfahren zur Herstellung eines Copolymers nach einem der Ansprüche 1 bis 11, dadurch gekennzeichnet, daß das Copolymer durch Kondensation von Milchsäure und Weinsäure hergestellt wird.

13) Verfahren zur Herstellung eines Copolymers nach Anspruch 12, dadurch gekennzeichnet, daß kleinere Mengen einer weiteren Hydroxycarbonsäure, eines Lactids oder eines Lactons zugefügt werden.

14) Verfahren zur Herstellung eines Copolymerisates nach einem der Ansprüche 12 bis 13, dadurch gekennzeichnet, daß die Polykondensationsreaktion in Gegenwart eines Katalysators durchgeführt wird.

15) Verfahren zur Herstellung eines Copolymers nach einem der Ansprüche 12 bis 14, dadurch gekennzeichnet, daß das entstehende Wasser aus dem Reaktionsgemisch während der Reaktion abdestilliert wird.

16) Verwendung eines Copolymers nach einem der Ansprüche 1 bis 11 als chirurgisches Hilfsmittel.

17) Verwendung eines Copolymers nach einem der Ansprüche 1 bis 11 als Träger oder Behältnis für therapeutische Mittel.

18) Verwendung eines Copolymers nach einem der Ansprüche 1 bis 11 als resorbierbares Implantat.

19) Verwendung eines Copolymers nach einem der Ansprüche 1 bis 11 als Träger oder Behältnis für Agrochemikalien, Insektizide oder Herbizide.

20) Verwendung eines Copolymers nach einem der Ansprüche 1 bis 11 als wasserlöslichen Schutzfilm.